(19) Europäisches Patentamt / European Patent Office / Office européen des brevets

(11) **EP 3 628 215 B1**

(12) **EUROPEAN PATENT SPECIFICATION**

(45) Date of publication and mention
of the grant of the patent:
**16.06.2021 Bulletin 2021/24**

(51) Int Cl.:
*A61B 5/00* *(2006.01)*     *A61B 5/021* *(2006.01)*
*A61B 5/349* *(2021.01)*     *A61B 5/389* *(2021.01)*

(21) Application number: **19209364.9**

(22) Date of filing: **30.03.2017**

(54) **METHOD FOR EXTRACTING FEATURE OF BIO-SIGNAL**

VERFAHREN ZUR EXTRAKTION EINES MERKMALS EINES BIOSIGNALS

PROCÉDÉ D'EXTRACTION DE CARACTÉRISTIQUE DE BIOSIGNAL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.07.2016 KR 20160092166**

(43) Date of publication of application:
**01.04.2020 Bulletin 2020/14**

(62) Document number(s) of the earlier application(s) in
accordance with Art. 76 EPC:
**17163720.0 / 3 272 279**

(73) Proprietor: **Samsung Electronics Co., Ltd.
Gyeonggi-do 16677 (KR)**

(72) Inventors:
- **PARK, Chang Soon
  Chungcheongbuk-do (KR)**
- **KWON, Ui Kun
  Gyeonggi-do (KR)**
- **KIM, Young Soo
  Seoul (KR)**

(74) Representative: **Grünecker Patent- und
Rechtsanwälte
PartG mbB
Leopoldstraße 4
80802 München (DE)**

(56) References cited:
WO-A1-2014/055797     US-A- 5 365 428
US-A1- 2012 108 987

**EP 3 628 215 B1**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

BACKGROUND

**1. Field**

Methods and apparatuses consistent with the exemplary embodiments disclosed herein relate to an apparatus and method for extracting a feature from a bio-signal in order to detect bio-information, and an apparatus for detecting bio-information using the method for extracting a feature.

**2. Description of Related Art**

In recent years, due to soaring medical costs, an aging population structure, and a lack of human resources for specialized medical services, research on IT-medical convergence technologies is being conducted, in which IT (information technology) and medical technology are combined. In particular, monitoring of the health condition of the human body is not being limited to being performed in a few fixed places such as hospitals but is expanding into mobile healthcare fields that monitor health conditions of users anywhere and anytime in everyday life such as in homes or offices. As representative examples of bio-signals that represent the health condition of individuals, there are ECG (electrocardiography) signals, PPG (photoplethysmography) signals, EMG (electromyography) signals, and the like, and a variety of bio-signal sensors have been developed to measure these signals in daily life. In particular, in the case of a PPG sensor, it is possible to estimate blood pressure of a human body by analyzing a pulse waveform that reflects a cardiovascular condition or the like.

According to research results related to PPG bio-signals, the overall PPG signals are a superposition of propagation waves starting from the heart toward the distal end portions of the body and reflection waves returning back from the distal end portions. Also, it is known that information through which the blood pressure can be estimated can be obtained by extracting various features related to the propagation waves or the reflection waves.

US 5 365 428 A discloses a device for reducing the number of ECG data sample points presented to a video display system. The device ensures that the maximum and minimum values of the original ECG signal are displayed. A collection of consecutive points is broken into sub-groups, which are each processed to produce representative amplitude values for each sub-group. The representative values are then displayed on the display screen.

It is the object of the present invention to provide an improved method and system for extracting a feature from a bio-signal.

This object is solved by the subject matter of the independent claims.

Embodiments are defined by the dependent claims.

This summary is provided to introduce a selection of concepts in a simplified form that are further described below in the detailed description. This summary is not intended to identify key features or essential features of the claimed subject matter, nor is it intended to be used as an aid in determining the scope of the claimed subject matter.

According to an aspect of an exemplary embodiment, there is provided an apparatus configured to extract a feature from a bio-signal, the apparatus including: a bio-signal acquirer configured to acquire the bio-signal; and a processor configured to analyze concavity and convexity of a waveform of the bio-signal in intervals of the bio-signal, and extract the feature from the bio-signal based on the analyzed concavity and the analyzed convexity.

The processor may include: a convex-to-concave difference (CCD) calculator configured to calculate a CCD value based on the concavity and convexity local minimum value in the intervals as a CCD value, and a feature extractor configured to extract the feature from the bio-signal based on the calculated CCD values.

The feature extractor may be configured to select a preset number of the intervals in descending order of the calculated CCD values, and extract the feature based on a local minimum point of the selected intervals.

The feature may include at least one of time information of the local minimum point and amplitude information of a bio-signal position corresponding to the time information.

The processor may further include an interval segmenter configured to segment the derived secondary differential signal into a preset number of the intervals.

The interval segmenter may be configured to segment a portion of the waveform from a local maximum point of the secondary differential signal to a next local minimum point as one of the intervals.

The interval segmenter may be configured to merge the segmented two or more intervals based on a difference between a local minimum value and a next local maximum value in the secondary differential signal.

According to an aspect of another exemplary embodiment, there is provided a method for extracting a feature from a bio-signal, the method including: acquiring the bio-signal; deriving a secondary differential signal of the bio-signal; analyzing concavity and convexity of a waveform of the secondary differential signal; and extracting the feature from the bio-signal based on the analyzed concavity and the analyzed convexity.

The analyzing may include calculating a difference between a local maximum value and a local minimum value in the intervals as a convex-to-concave difference (CCD) value, and the extracting may include extracting the feature from the bio-signal based on the calculated CCD values.

The extracting of the feature may include selecting a preset number of intervals in descending order of the calculated CCD values, and extracting the feature based on a local minimum point of each of the selected intervals.

The method may further include segmenting the derived secondary differential signal into a preset number of the intervals.

The segmenting may include segmenting an interval from a local maximum point of the secondary differential signal to a next local minimum point as a single one of the intervals.

The method may further include merging the segmented two or more intervals based on a difference between a local minimum value and a next local maximum value in the secondary differential signal.

According to an aspect of another exemplary embodiment, there is provided an apparatus configured to detect bio-information, the apparatus including: a sensor configured to irradiate a subject with light and detect the light reflected by the subject, to measure a bio-signal; and a processor configured to extract a feature from the bio-signal based on concavity and convexity of a waveform of the bio-signal in intervals of the bio-signal when the bio-signal is measured, and detect bio-information based on the extracted feature.

In response to measuring the bio-signal, the processor may be configured to derive a secondary differential signal of the measured bio-signal, and segment the derived secondary differential signal into the intervals based on a local maximum point and a local minimum point of the derived secondary differential signal.

The processor may be configured to select a preset number of intervals from the segmented intervals, extract time information of a local minimum point of the selected intervals and amplitude information of a bio-signal position corresponding to the time information, and detect the bio-information by combining at least two pieces of the extracted amplitude information.

The processor may be configured to monitor a user's health condition based on the bio-information, and generate additional information according to the health condition.

The bio-information may include blood pressure information, and the additional information may include warning information.

The apparatus may further include: a display configured to display the detected bio-information and the additional information.

Other features and aspects will be apparent from the following detailed description, the drawings, and the claims.

BRIEF DESCRIPTION OF THE DRAWINGS

FIG. 1 is a block diagram illustrating an apparatus for extracting a feature of a bio-signal according to an exemplary embodiment;

FIGS. 2A, 2B, and 2C are diagrams for describing a general method for extracting a feature from a bio-signal;

FIG. 3 is a block diagram illustrating an exemplary embodiment of a processing unit of the apparatus for extracting a feature of a bio-signal according to the exemplary embodiment of FIG. 1;

FIG. 4 is a diagram for describing a method for analyzing a waveform of a bio-signal, which is performed by the processing unit according to the exemplary embodiment of FIG. 3;

FIG. 5 is a block diagram illustrating another exemplary embodiment of the processing unit of the apparatus for extracting a feature of a bio-signal according to the exemplary embodiment of FIG. 1;

FIGS. 6A, 6B, 6C, 6D, and 6E are diagrams for describing a method for analyzing a waveform of a bio-signal, which is performed by the processing unit according to the exemplary embodiment of FIG. 5;

FIGS. 7A and 7B are diagrams for describing a method for segmenting intervals of a waveform of a bio-signal, which is performed by the processing unit according to the exemplary embodiment of FIG. 5;

FIG. 8 is a flowchart illustrating a method for extracting a feature of a bio-signal according to an exemplary embodiment;

FIG. 9 is a flowchart illustrating a method for extracting a feature of a bio-signal according to another exemplary embodiment;

FIG. 10 is a block diagram illustrating an apparatus for detecting bio-information according to an exemplary embodiment;

FIG. 11 is a detailed block diagram illustrating a processing unit of an apparatus for detecting bio-information according to an exemplary embodiment; and

FIG. 12 is a flowchart illustrating a method for detecting bio-information according to an exemplary embodiment.

Throughout the drawings and the detailed description, unless otherwise described, the same drawing reference numerals should be understood as referring to the same elements, features, and structures. The relative size and depiction of these elements may be exaggerated for clarity, illustration, and convenience.

DETAILED DESCRIPTION

Advantages and characteristics of the technical disclosure and methods for achieving them should become apparent with reference to exemplary embodiments described in detail below in addition to the accompanying drawings. However, the scope of the disclosure is not limited to the exemplary embodiments which will be described below but may be implemented in various forms. Throughout the specification, like elements refer to like reference numerals.

It will be understood that, although the terms first, second, etc., may be used herein to describe various elements, these elements should not be limited by these terms. These terms are only used to distinguish one element from another. As used herein, the singular forms "a", "an" and "the" are intended to include the plural forms as well, unless the context clearly indicates otherwise. In addition, when a unit "comprises" an element, the unit does not exclude another element but may further comprise another element unless the context clearly indicates otherwise. In addition, terms such as "...unit", "module", and the like used herein indicate a unit for performing at least one function or operation and may be implemented by hardware or software or a combination thereof.

Hereinafter, exemplary embodiments of an apparatus and method for extracting a feature of a bio-signal and an apparatus for detecting bio-information will be described in detail with reference to the accompanying drawings. For convenience of description, terms or expressions such as concavity, convexity, "downward convex", "upward convex", "downward concave", "upward concave", etc., may be used in various ways and in various combinations. However, such terms and expressions may be defined with respect to a case in which a waveform of a bio-signal is downward convex. Thus, convexity may be described as a characteristic in which the waveform is downward convex, and the concavity may be described as a characteristic in which the waveform is upward convex. In addition, "downward convex" may have the same meaning as a waveform that is upward concave, and "upward convex" may have the same meaning as a waveform that is downward concave.

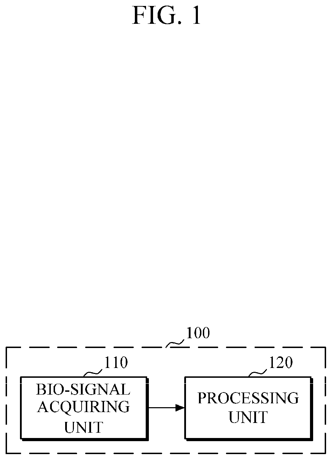

FIG. 1 is a block diagram illustrating an apparatus for extracting a feature of a bio-signal according to an exemplary embodiment. The apparatus 100 for extracting a feature of a bio-signal may be installed in the form of a hardware or software module in a terminal such as a wearable device, a smart phone, a tablet PC, a desktop PC, a notebook PC, etc., or a medical device such as a blood pressure measurement device, etc. Alternatively, the apparatus 100 for extracting a feature of a bio-signal may be implemented as an independent hardware device and, in this case, may be utilized for research purposes for analyzing the waveform of a bio-signal constituted of a sum of a plurality of constituent pulses. However, the apparatus 100 for extracting a feature of a bio-signal is not limited thereto, and may be variously modified and implemented, depending on the utilization purposes.

Referring to FIG. 1, an apparatus 100 for extracting a feature of a bio-signal includes a bio-signal acquiring unit 110 (e.g., bio-signal acquirer) and a processing unit 120 (e.g., processor). The bio-signal acquiring unit 110 and the processing unit 120 may be implemented by one or more circuits, processors, memories, or a combination thereof.

The bio-signal acquiring unit 110 may acquire a bio-signal of a subject to be used for extracting bio-information of the subject and transmit the acquired bio-signal to the processing unit 120. The bio-information may be blood pressure, or the bio-signal may be an ECG (electrocardiography) signal, a PPG (photoplethysmography) signal, an EMG (electromyography) signal, or the like.

Hereinafter, for convenience of description, description will be made assuming that the PPG signal (hereinafter, referred to as "a pulse wave signal") is the bio-signal and the blood pressure is the bio-information. However, bio-signal and bio-information are not to be construed as being limited to the PPG signal and the blood pressure.

As an example, the bio-signal acquiring unit 110 may control a sensor according to a predetermined control signal and receive a pulse wave signal of the subject from the corresponding sensor. The control signal may be generated by a control module implemented in the apparatus 100 for extracting a feature. The control module may be implemented as one function of the processing unit 120 and generate the control signal based on a user's input. The sensor may drive a light source to irradiate the subject with light when receiving the control signal and acquire the pulse wave signal by detecting light which has been scattered or reflected from the subject and then returned. When receiving the pulse wave signal, the sensor may transmit the acquired pulse wave signal to the bio-signal acquiring unit 110.

The sensor may be mounted in the apparatus 100 for extracting a feature or in other separate hardware devices. When the sensor is mounted in a different hardware device, the sensor and the apparatus 100 for extracting a feature may be equipped with a communication module for enabling wired and wireless communication and connected to each other through the communication module, thereby transmitting and receiving the control signal, the pulse wave signal, and the like.

As another example, the bio-signal acquiring unit 110 may receive bio-signals, for example, from other external devices. The other external devices may be devices which are installed in a medical institution or the like to measure a variety of bio-signals for detecting bio-information and store the measured bio-signals. The other external devices may transmit the bio-signal to the bio-signal acquiring unit 110 when it is necessary or desired to extract a feature from the measured bio-signal.

The processing unit 120 receives the bio-signal from the bio-signal acquiring unit 110. The processing unit 120 may perform pre-processing such as filtering for removing noise from the received bio-signal. For example, the processing unit 120 may extract the feature by analyzing the waveform of the bio-signal in every constant interval unit. To this end, the processing unit 120 may segment the waveform of the bio-signal into predetermined intervals and extract the feature in consideration of concavity and convexity for each of the segmented intervals. According to an exemplary embodiment, the feature may be information such as time, amplitude, or a combination thereof, but is not limited thereto. The feature may include a variety of information according to the type and characteristics of the bio-signal.

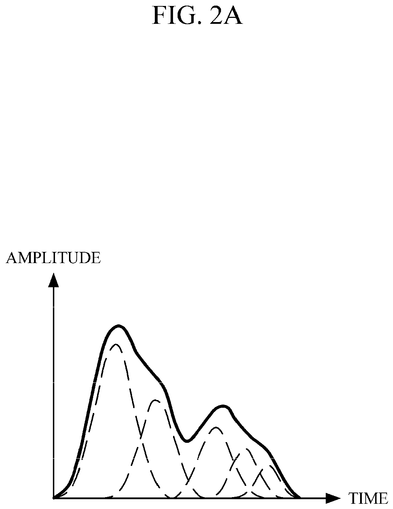

Hereinafter, a variety of exemplary embodiments of the processing unit 120 that extracts the feature from the bio-signal will be described with reference to FIGS. 2A to 7.

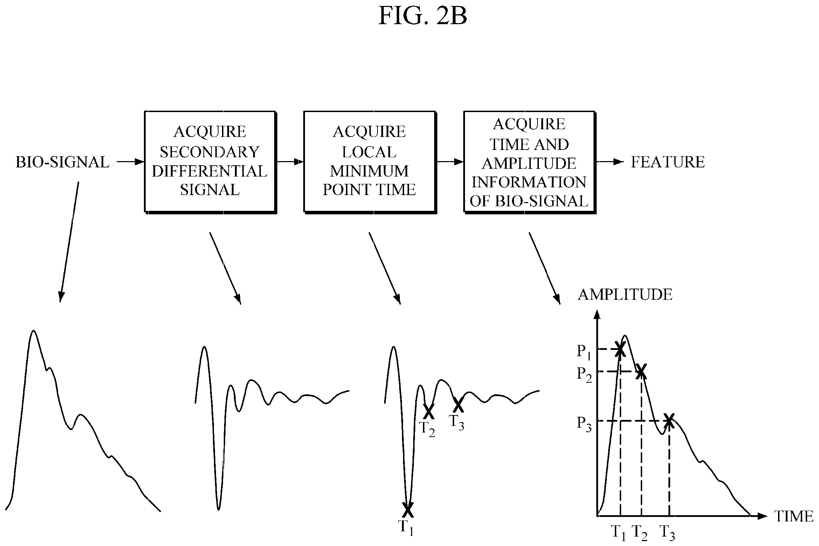

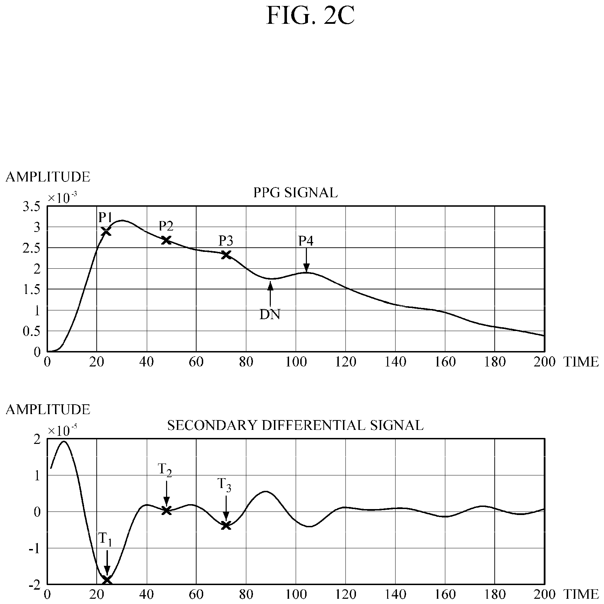

FIGS. 2A, 2B, and 2C are diagrams for describing a general method for extracting a feature from a bio-signal.

FIG. 2A shows a pulse wave signal (a solid line) and constituent pulses (dotted lines) constituting the pulse wave signal. As shown in FIG. 2A, it can be seen that a single waveform of the pulse wave signal is formed such that five constituent pulses are superposed. According to an exemplary embodiment, by appropriately combining information such as time, amplitude, and the like corresponding to the constituent pulses, it is possible to extract a feature having high correlation with blood pressure. In general, as the constituent pulses used for estimating blood pressure, pulses up to the third pulse are mainly used. Usually, finding pulses after the third pulse is difficult due to noise and they have a low correlation with estimation of the blood pressure. Also, pulses after the third pulse are not even observed for some people.

FIG. 2B shows a general procedure of extracting a feature using a bio-signal. In general, an apparatus for extracting a feature from a bio-signal may search for a local minimum point in a secondary differential signal of the bio-signal. Next, the apparatus may acquire, as characteristic points, time information $T_1$, $T_2$, and $T_3$ corresponding to the local minimum point and amplitude information $P_1$, $P_2$, and $P_3$ of the bio-signal corresponding to the time information $T_1$, $T_2$, and $T_3$ and extract the feature for detecting bio-information using the acquired characteristic points. According to an exemplary embodiment, the local minimum point refers to a point that has a form in which the corresponding signal is reduced and then increased again with respect to a specific point when some intervals of the secondary differential signal are observed. This secondary differential signal may be referred to as a downward convex form.

FIG. 2C shows a pulse wave signal (upper graph in FIG. 2C) and a signal (lower graph in FIG. 2C) obtained by secondarily differentiating the pulse wave signal. According to an exemplary embodiment, the pulse wave signal is obtained with a sampling frequency of 250 Hz, and a horizontal axis corresponds to a sample index that increases proportionally with time.

Assuming that the number of characteristic points used for extracting a feature is 3, when a general method for extracting a feature is used for such a pulse wave signal, three time positions $T_1$, $T_2$, and $T_3$ corresponding to the local minimum point of the secondary differential signal and amplitude positions $P_1$, $P_2$, and $P_3$ of the pulse wave signal corresponding to the time positions $T_1$, $T_2$, and $T_3$ are obtained. However, the amplitude positions which should be actually acquired from this pulse wave signal are $P_1$, $P_3$, and $P_4$. The acquisition of incorrect amplitude positions is caused by a very slightly upward convex form that is generated due to a non-ideal change in the waveform in the position $P_2$ that makes the position $T_2$ become the local minimum point in the waveform of the secondary differential signal.

When the bio-signals are generally classified into a front portion corresponding to systole of the heart and a rear portion corresponding to diastole, the third amplitude generally corresponds to the top of the upward convex form in the diastole. Also, there is a downward convex point DN, which is physiologically called a dicrotic notch, at the time immediately prior to the third amplitude position. In Fig. 2C the amplitude position $P_4$ which is the top of the upward convex form at a point in time next to a point in time corresponding to the point DN at which the dicrotic notch appears should actually be the third amplitude position. In addition, an actual position of the second amplitude should be $P_3$ before the dicrotic notch.

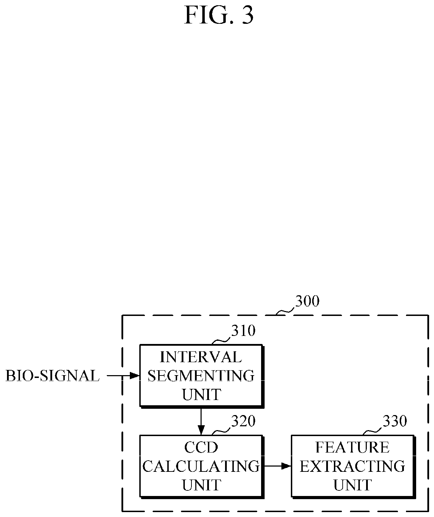

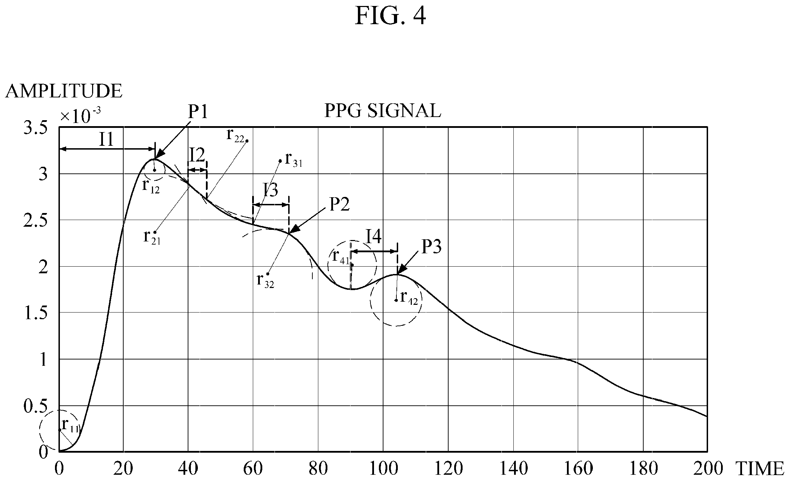

FIG. 3 is a block diagram illustrating an exemplary embodiment of the processing unit 120 of the apparatus 100 for extracting a feature of a bio-signal according to the exemplary embodiment of FIG. 1, and FIG. 4 is a diagram for describing a method for analyzing the waveform of bio-signal, which is performed by a processing unit 300 according to the exemplary embodiment of FIG. 3.

Referring to FIG. 3, the processing unit 300 may include an interval segmenting unit 310 (e.g., interval segmenter), a CCD (convex-to-concave difference) calculating unit 320 (e.g., CCD calculator), and a feature extracting unit 330 (e.g., feature extractor).

The interval segmenting unit 310 segments the waveform of the acquired bio-signal into predetermined intervals. According to an exemplary embodiment, the interval segmenting unit 310 may segment the waveform of the bio-signal to obtain a predetermined number of characteristic points. The predetermined number may be set as 3 when the blood pressure is measured from the pulse wave signal. However, the predetermined number is not limited thereto and may be adjustable according to the kind of the bio-information and bio-signal to be detected, characteristics of the waveform of the bio-signal, functions of the apparatus 100 or a device to which the apparatus 100 is applied, and the like. In addition, the number of intervals to be segmented may be set in consideration of complexity and performance of the apparatus, accuracy of the bio-information to be detected, and the like.

Meanwhile, the interval segmenting unit 310 may adaptively adjust the predetermined number of intervals to be segmented according to the shape of the waveform of the acquired bio-signals. For example, a condition for a last interval to be segmented may be set in advance based on physiological characteristics of each of the constituent pulses, and when the last interval to be segmented does not satisfy the corresponding condition, segmentation may be repeatedly performed until the corresponding condition is satisfied.

In addition, the interval segmenting unit 310 may allow an interval having a small degree to which the waveform becomes downward convex and then upward convex to be merged to a previous interval.

The CCD calculating unit 320 may analyze a degree to which the waveform becomes downward convex and then upward convex for each interval when the waveform of the bio-signal is segmented into constant intervals, thereby calculating a CCD value. The CCD calculating unit 320 may define a downward convex portion as a positive (+) value in the bio-signal waveform and calculate a difference in a downward convex degree at a specific point in time in each interval and a point in time immediately after the specific point in time as a CCD value of the corresponding interval. For example, the CCD calculating unit 320 may calculate a degree C1 to which the waveform of the entire bio-signal is downward convex at a start point in time of each interval and a degree C2 to which the waveform is downward convex at an end point in time, and obtain a value obtained by subtracting C2 from C1 as a CCD value.

For example, the CCD calculating unit 320 may calculate a curvature at a start point in time and an end point in time of each separated interval, and calculate a CCD based on the calculated curvature at each interval. The CCD calculating unit 320 may calculate, as a CCD of the corresponding interval, a value obtained by subtracting the curvature at the end point in time from the curvature at the start point in time of the corresponding interval. At this point, assuming that the waveform of the bio-signal is downward convex is defined as a positive (+) value, when the start point in time or the end point in time is upward convex, a negative (-) curvature obtained by multiplying a value calculated at the corresponding point in time by -1 may be used in calculation of the CCD. According to an exemplary embodiment, the curvature is a rate of change indicating the degree of bending of a curved line or a curved surface, and when it is assumed that a minute portion of the curved line is a circular arc, a radius of the circular arc may be called a radius of curvature, and the inverse number of the radius of curvature may be called a curvature.

Referring to FIG. 4, a radius of curvature at a start point in time of a first interval I1 is $r_{11}$, so that a curvature $k_{11}$ is $1/r_{11}$, and a radius of curvature at an end point in time is $r_{12}$, so that a curvature $k_{12}$ is $1/r_{12}$. Accordingly, the waveform of the bio-signal at the end point in time of the first interval I1 is upward convex, and thus a $CCD_1$ of the first interval I1 is calculated as being $k_{11}-(-k_{12})$ using a value obtained by multiplying the curvature $k_{12}$ by -1. The waveform of the bio-signal at a start point in time is upward convex, and thus a $CCD_2$ of a second interval I2 is calculated as being $(-k_{21})-(k_{22})$ using a negative value obtained by multiplying a curvature $k_{21}$ at the start point in time by -1. In the same manner, a $CCD_3$ of a third interval I3 is calculated as being $k_{31}-(-k_{32})$, and a $CCD_4$ of a fourth interval I4 is calculated as being $k_{41}-(-k_{42})$. The curvatures $k_{11}$, and $k_{12}$ of the first interval I1 are larger than those of the other intervals 12, 13, and 14, so that the $CCD_1$ has the largest value, and the curvatures $k_{21}$ and $k_{22}$ at the start point in time and the end point in time of the second interval I2 are small and the start point in time is upward convex, so that the $CCD_2$ is a negative value which is the smallest value. In addition, the fourth interval I4 has a larger curvature than that of the third interval 13, and thus the $CCD_4$ of the fourth interval I4 is calculated to be larger than the $CCD_3$ of the third interval 13.

The feature extracting unit 330 may select three intervals I1, 13, I4 based on the CCD value of each interval and extract a feature from the bio-signal of the selected intervals. For example, the feature extracting unit 330 may select the same number of intervals as the predetermined number of characteristic points in descending order of the calculated CCD values. In addition, the feature extracting unit 330 may extract time at a specific point of each of the selected intervals, for example, a last point of each of the selected intervals and amplitudes P1, P2 and P3 of the bio-signals corresponding to the time, as the characteristic points. In addition, the feature extracting unit 330 may extract the feature F used for detecting the bio-information using the extracted characteristic points as shown in Equation 1 below.

Referring to FIG. 4, since the CCD values are calculated in order of the intervals I1, 14, 13, and I2 in this manner, when assuming that the predetermined number of the characteristic points is 3, the feature extracting unit 330 may select the remaining three intervals I1, 13, and 14, that is, except for the second interval 12, according to the descending order of the CCD value. The feature extracting unit 330 may acquire amplitudes P1, P2, and P3 as the characteristic points from the selected intervals I1, 13, and I4 and combine the amplitudes P1, P2, and P3 as shown in the following Equation 1 and utilize the combined amplitudes as a feature F for estimating the blood pressure.

[Equation 1]

$$F=(P1+P2)/P3$$

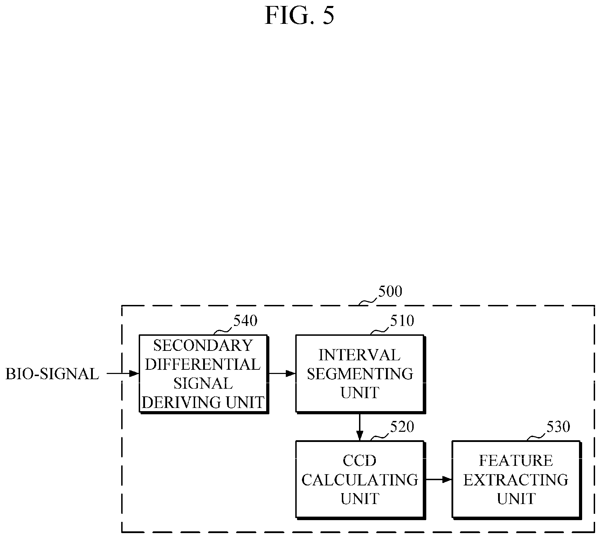

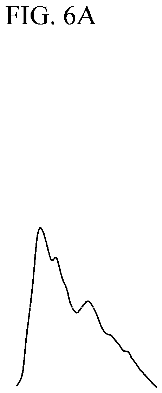

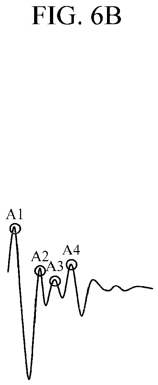

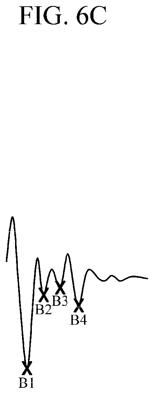

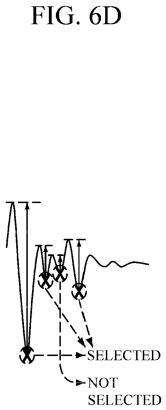

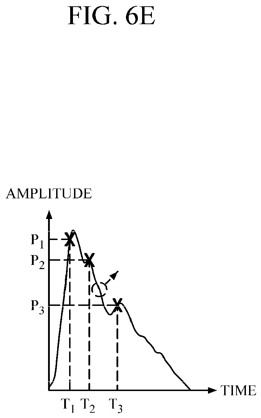

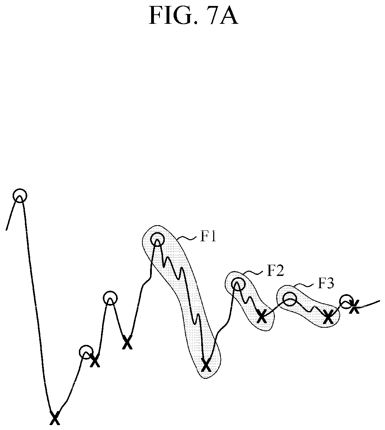

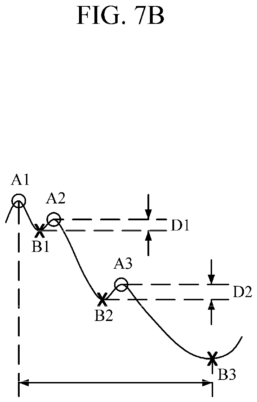

FIG. 5 is a block diagram illustrating another exemplary embodiment of the processing unit 120 of the apparatus 100 for extracting a feature of a bio-signal according to the exemplary embodiment of FIG. 1, FIG. 6 is a diagram for describing a method for analyzing a waveform of a bio-signal, which is performed by a processing unit 500 according to the exemplary embodiment of FIG. 5, and FIG. 7 is a diagram for describing a method for segmenting intervals of a waveform of a bio-signal, which is performed by the processing unit 500 according to the exemplary embodiment of FIG. 5.

Referring to FIG. 5, the processing unit 500 may include an interval segmenting unit 510 (e.g., interval segmenter), a CCD calculating unit 520 (e.g., CCD calculator), a feature extracting unit 530 (e.g., feature extractor), and a secondary differential signal deriving unit 540 (e.g., secondary differential signal deriver).

When bio-signals are acquired, the secondary differential signal deriving unit 540 derives a secondary differential signal by secondarily differentiating the acquired bio-signals. For example, when assuming that the secondary differential signal is a digital sample index in which a time value t is incremented by 1 in a bio-signal waveform function g(t), the secondary differential signal deriving unit 540 may obtain a primary differential signal g'(t) and a secondary differential signal g"(t) using the following Equations 2 and 3.

[Equation 2]

$$g'(t)=g(t)-g(t-1)$$

[Equation 3]

$$g''(t)=g'(t)-g'(t-1)$$

The interval segmenting unit 510 may segment the waveform of the secondary differential signal into predetermined intervals based on a local maximum point and a local minimum point. At this point, the number of intervals to be segmented may be set to be larger than or equal to the number of characteristic points desired to be acquired. The number of characteristic points may be set in advance based on the type of bio-information and bio-signals to be detected. The number of intervals to be segmented and the number of characteristic points to be acquired may be set in advance as the appropriate number in consideration of the complexity and performance of the apparatus, the type and accuracy of the bio-information to be detected, the type of the acquired bio-signals, and the like. According to an exemplary embodiment, the local maximum point and the local minimum point may respectively refer to a point having the largest convexity and a point having the largest concavity in a local interval of a secondary differential signal waveform when the secondary differential signal waveform is defined with respect to a downward convex degree.

The CCD calculating unit 520 calculates a CCD value in each interval of the segmented secondary differential signal. For example, the CCD calculating unit 520 may obtain a difference between a local maximum value $LMax_i$ and a local minimum value $LMin_i$ in a segmented interval i through the following Equation 4, and calculate the resulting $CCD_i$ as a CCD value of the interval i. According to an exemplary embodiment, the local maximum value may be a secondary differential value of the local maximum point, and the local minimum value may be a secondary differential value of the local minimum point.

[Equation 4]

$$CCD_i = LMax_i - LMin_i$$

The feature extracting unit 530 extracts a feature from a bio-signal based on the CCD value calculated by the CCD calculating unit 520. When the CCD values are calculated for each of the intervals, the feature extracting unit 530 may align the CCD values in descending order, select intervals corresponding to the predetermined number of characteristic points according to the descending order of the CCD value, and extract characteristic points from each of the selected intervals. In addition, the feature extracting unit 530 may extract a feature used for detecting the bio-information by utilizing the extracted characteristic points.

For example, the feature extracting unit 530 may acquire one piece of time information and amplitude information of the bio-signal corresponding to the time information from each of the selected intervals as the characteristic points. According to an exemplary embodiment, time at the local minimum point of each of the selected intervals may be acquired as the time information, and amplitude of a bio-signal corresponding to the time may be acquired as the amplitude information. However, the characteristic points may be acquired in consideration of information of the local maximum point. For example, an average, an intermediate value, or the like between time at the local maximum point and time at the local minimum point may be acquired as the time information. The feature extracting unit 530 may extract a feature by combining the amplitude information acquired, as shown in Equation 1.

With reference to FIGS. 6A, 6B, 6C, 6D, and 6E, a procedure of extracting a feature performed by the processing unit 500 will be described. At this point, it is assumed that the number of characteristic points desired to be acquired is 3 and the number of intervals to be segmented is 4. First, FIG. 6A shows a bio-signal which is acquired. When a bio-signal is acquired, the secondary differential signal deriving unit 540 may derive a secondary differential signal by differentiating the bio-signal. Each of FIGS. 6B, 6C, and 6D show a secondary differential signal, and the interval segmenting unit 510 may acquire local maximum points A1, A2, A3, and A4 and local minimum points B1, B2, B3, and B4 by searching for the secondary differential signal. In addition, when the local maximum points A1, A2, A3, and A4 and the local minimum points B1, B2, B3, and B4 are acquired, the interval segmenting unit 510 may segment a corresponding interval from the first local maximum point A1 to the following local minimum point B1 into a first interval A1-B1 and, in the same manner as in the first interval A1-B1, segment, in the stated order, a second interval A2-B2, a third interval A3-B3, and a fourth interval A4-B4.

The CCD calculating unit 520 calculates a CCD value with respect to each of the segmented intervals. The CCD calculating unit 520 may calculate a value, obtained by subtracting a secondary differential value of the local minimum point from a secondary differential value of the local maximum point of each interval, as the CCD value of the corresponding interval.

The feature extracting unit 530 may select three intervals corresponding to the number of characteristic points to be acquired based on the calculated CCD value of each interval as shown in FIG. 6D. At this point, a first interval, a fourth interval, and a second interval may be selected in descending order of the CCD value, for example, in descending order of a difference between the local maximum value and the local minimum value. In addition, the feature extracting unit 530 may acquire time information $T_1$, $T_2$, and $T_3$ corresponding to the local minimum point from the selected three intervals and amplitude information $P_1$, $P_2$, and $P_3$ of positions corresponding to the time information $T_1$, $T_2$, and $T_3$ from a bio-signal, as shown in FIG. 6E.

The interval segmenting unit 510 may adaptively adjust the number of intervals to be segmented in consideration of the type of a bio-signal waveform, that is, physiological characteristics of each constituent pulse. For example, in a case in which the number of intervals to be segmented is 4, which is initially set in advance, when the final fourth interval does not satisfy a preset condition, the interval segmenting unit 510 may continue to segment until the final fourth interval satisfies the condition. According to an exemplary embodiment, the preset condition may be that the local maximum value and the local minimum value which are utilized in calculating the CCD value should have a positive value and a negative value, respectively, but exemplary embodiments are not limited thereto.

When segmenting a secondary differential signal waveform into constant intervals, the interval segmenting unit 510 may merge two or more intervals into a single interval in consideration of the characteristics of the waveform and the like. When a high-frequency component which irregularly fluctuates due to a non-ideal cause is contained in the acquired bio-signal, the secondary differential signal may unstably fluctuate. In this case, the interval segmenting unit 510 may merge two or more segmented intervals on the basis of a fluctuation of the waveform at two or more points of the segmented interval in the secondary differential signal.

For example, referring to FIG. 7A, FIG. 7A shows the presence of severe fluctuation intervals F1, F2, and F3 in a secondary differential signal due to an unstable waveform. When the intervals F1, F2, and F3 in which the secondary differential signal severely fluctuates are present in this manner, the interval segmenting unit 510 may merge the severe fluctuation intervals F1, F2, and F3 into a single interval according to predetermined criteria.

FIG. 7B is an example describing a method for merging fluctuation intervals in the secondary differential signal. When segmenting first local maximum point A1 and local minimum point B1, second local maximum point A2 and local minimum point B2, and third local maximum point A3 and local minimum point B3, respectively as a first interval, a second interval, and a third interval in the stated order, the interval segmenting unit 510 may determine whether to merge an interval to be currently segmented into a previous interval.

For example, when segmenting the first interval and then segmenting the second interval, the interval segmenting unit 510 may calculate a difference between a secondary differential value of the local maximum point A2 of the second interval and a secondary differential value of the local minimum point B1 of the first interval as fluctuation D1 of the second interval. If the calculated fluctuation D1 of the second interval is smaller than a preset reference value, the interval segmenting unit 510 may merge the second interval into the first interval.

Similarly, when segmenting the third interval, the interval segmenting unit 510 may calculate a difference between a second differential value of the local maximum point A3 of the third interval and a secondary differential value of the local minimum point B2 of the second interval as fluctuation D2 of the third interval. Next, when the fluctuation D2 of the third interval is smaller than the reference value, the interval segmenting unit 510 may merge the third interval into the second interval.

When the fluctuation D1 of the second interval and the fluctuation D2 of the third interval are all smaller than a threshold value, the first interval, the second interval, and the third interval may be merged into a single interval, and a local maximum point and a local minimum point of the merged interval are respectively the local maximum point A1 of the first interval and the local minimum point B3 of the last interval before the corresponding intervals are merged.

According to an exemplary embodiment, the reference value which is used to determine whether to merge the corresponding intervals may be appropriately set in consideration of a measurement condition of the bio-signal, the performance of the apparatus, and the like. For example, the reference value may be a value obtained by multiplying two values: a first value for considering a case of different measurement value depending on the measurement condition of the bio-signal and a value set in advance in consideration of the performance of the apparatus, or the like. According to an exemplary embodiment, the first value may be a CCD value which is calculated with respect to a first constituent pulse which is acquired in a relatively stable manner but is not limited thereto.

According to the disclosed exemplary embodiment, the intervals may be segmented and merged by considering the fluctuation of the waveform while considering concavity and convexity by analyzing the waveform of the bio-signal or the secondary differential signal for every constant-interval unit, so that it is possible to reduce an error that occurs when the characteristic points are extracted in consideration of only the local minimum point from the bio-signal which is non-ideally changed, as described above with reference to FIGS. 2A to 2C.

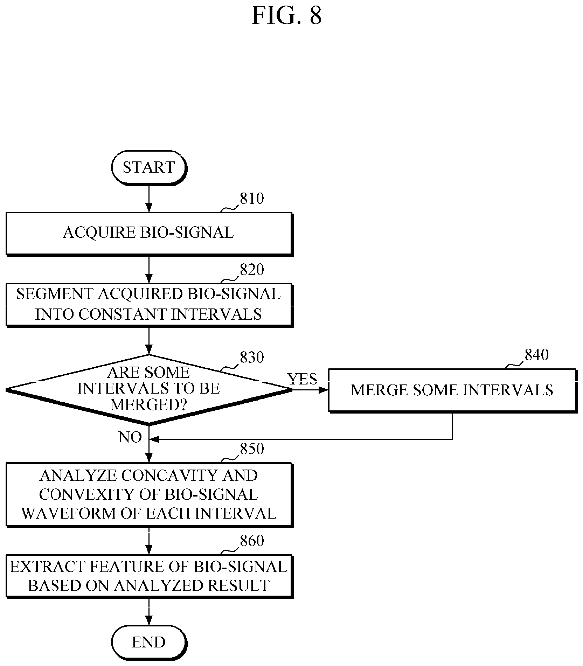

FIG. 8 is a flowchart illustrating a method for extracting a feature of a bio-signal according to an exemplary embodiment.

FIG. 8 is an exemplary embodiment of a method performed by the apparatus 100 for extracting a feature. The apparatus 100 has been described in detail with reference to FIGS. 1 to 4 and thereby will be simply described in order to avoid repeated description.

First, in operation 810, the apparatus 100 for extracting a feature may acquire a bio-signal. The apparatus 100 for extracting a feature may control a sensor for measuring a bio-signal which is mounted in the apparatus 100 or connected, either by a wire or wirelessly, to the apparatus 100, for example, a PPG sensor according to a predetermined control signal, and thereby acquire a pulse wave signal. Alternatively, the apparatus 100 for extracting a feature may receive a bio-signal for detecting bio-information from other devices which store the bio-signal.

Next, in operation 820, the apparatus 100 for extracting a feature may segment the acquired bio-signal into constant intervals. At this point, the number of intervals to be segmented may be set to be larger than or equal to the preset number of characteristic points. In addition, the number of intervals to be segmented may be set according to the type of the bio-signal, the type and accuracy of bio-information to be detected, the performance of the apparatus, and the like and adaptively adjusted according to the type of the waveform of the bio-signal.

Next, the apparatus 100 for extracting a feature may determine whether some segmented intervals should be merged in operation 830 and merge the interval that should be merged into a previous interval in operation 840. The apparatus 100 for extracting a feature may determine whether a specific interval should be merged into the previous interval on the basis of fluctuation of a waveform of each interval. For example, the apparatus 100 for extracting a feature may calculate fluctuation of a waveform of a specific interval, compare the calculated fluctuation and a predetermined reference value, and decide to merge the specific interval into the previous interval when the fluctuation is smaller than the predetermined reference value.

Operations 820 to 840 may be performed sequentially or concurrently. For example, operation 830 and operation 840 need not be performed after all intervals are segmented in operation 820 and may be performed whenever a single interval is segmented in operation 820.

Next, in operation 850, the apparatus 100 for extracting a feature may analyze concavity and convexity of the waveform of the bio-signal in the segmented intervals. For example, the apparatus 100 for extracting a feature may calculate a CCD value indicating a degree to which the waveform of the bio-signal is downward convex and then upward convex in each interval. According to an exemplary embodiment, when a case in which the waveform of the bio-signal is downward convex is defined as a positive (+) value, a difference in a downward convexity at a start point in time and an end point in time of each interval may be calculated as a CCD value of the corresponding interval.

Next, in operation 860, the apparatus 100 for extracting a feature may extract a feature from the bio-signal based on the analysis result. For example, the apparatus 100 for extracting a feature may select intervals corresponding to the number of characteristic points to be extracted in descending order of the calculated CCD values, and extract the characteristic points from each of the selected intervals. Next, the apparatus 100 for extracting a feature may extract a feature used for detecting bio-information using the extracted characteristic points. The characteristic points may include time information and amplitude information, and the feature used for detecting bio-information may be extracted by combining two or more characteristic points, for example, amplitude information.

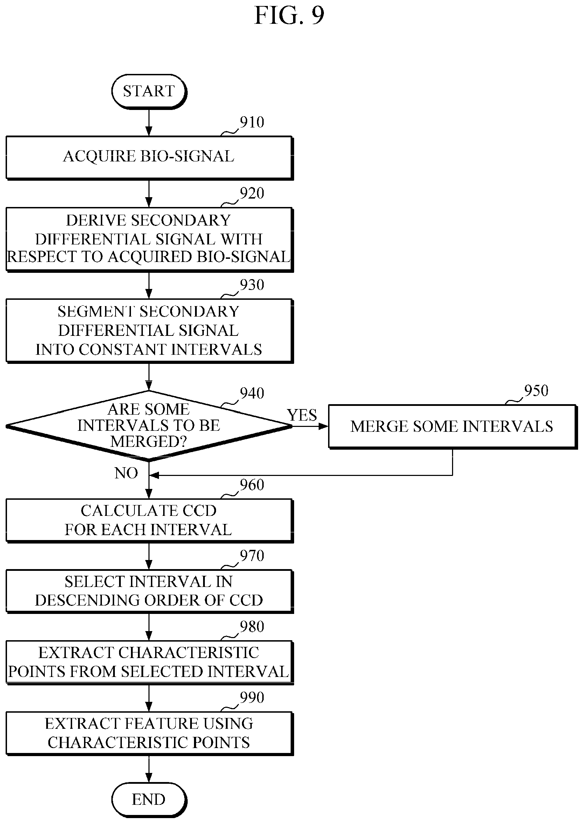

FIG. 9 is a flowchart illustrating a method for extracting a feature of a bio-signal according to another exemplary embodiment.

FIG. 9 is another exemplary embodiment of a method performed by the apparatus 100 for extracting a feature. The apparatus 100 has been described in detail with reference to FIGS. 1, 5, and 7, and thereby will be simply described in order to avoid repeated description.

First, in operation 910, the apparatus 100 for extracting a feature may acquire a bio-signal in order to detect bio-information such as blood pressure or the like. At this point, the bio-signal may be a pulse wave signal or may be various other types of bio-signals, as described above.

Next, in operation 920, the apparatus 100 for extracting a feature may derive a secondary differential signal by secondarily differentiating the acquired bio-signal. According to an exemplary embodiment, the secondary differential signal may be derived through the above-described Equations 2 and 3.

Next, in operation 930, the apparatus 100 for extracting a feature may segment the derived secondary differential signal into constant intervals. At this point, the apparatus 100 for extracting a feature may segment the secondary differential signal into constant intervals based on a local maximum point and a local minimum point in the waveform of the secondary differential signal. In addition, the apparatus 100 for extracting a feature may adaptively adjust the preset number of intervals to be segmented in consideration of the type of a bio-signal waveform, that is, physiological characteristics of each constituent pulse.

Next, in operation 940, the apparatus 100 for extracting a feature may determine whether to merge some intervals of the segmented constant intervals. For example, when a high-frequency component which irregularly shakes due to a non-ideal cause is contained in the acquired bio-signal, the secondary differential signal may unstably shake. In this manner, in a case in which a feature is extracted from the acquired bio-signal using a general method, a searching error such as a characteristic point being extracted at an inaccurate point may occur. In order to solve this problem, the apparatus 100 for extracting a feature may calculate a difference between a local maximum value of the segmented interval and a local minimum value of a previous interval as a fluctuation and determine whether to merge two or more segmented intervals based on the calculated fluctuation. That is, the apparatus 100 for extracting a feature may compare the calculated fluctuation and a preset reference value with respect to a specific interval and decide to merge the specific interval into a previous interval when the fluctuation is smaller than the reference value.

Next, in operation 950, the apparatus 100 for extracting a feature may merge some intervals determined to be merged into a previous interval. When two or more consecutive intervals are merged through the above-described process, a local maximum point and a local minimum point of the merged interval may be respectively a local maximum point of the first interval and a local minimum point of the last interval before the corresponding intervals are merged.

Meanwhile, operations 930 to 950 may be performed sequentially or concurrently. For example, operation 940 and operation 950 may be sequentially performed after all intervals are segmented in operation 930. Alternatively, when a single interval is segmented in operation 930, operations 940 and 950 may be immediately performed, and operations 930 to 950 may be repeatedly performed until the operations reach the number of intervals to be segmented.

Next, in operation 960, the apparatus 100 for extracting a feature may calculate a CCD value for each interval to be segmented. At this point, the CCD value may be a difference between a local maximum value and a local minimum value of each interval but is not limited thereto.

Next, in operation 970, when the CCD values are calculated, the apparatus 100 for extracting a feature may align the CCD values in descending order, and select intervals corresponding to the number of characteristic points to be acquired according to descending order of the CCD values.

Next, the apparatus 100 for extracting a feature may extract characteristic points from the selected intervals in operation 980 and extract a feature for detecting bio-information using the extracted characteristic points in operation 990. For example, the apparatus 100 for extracting a feature may acquire a time position corresponding to a local minimum point in each of the selected intervals as time information of the characteristic points and acquire amplitude of a position corresponding to the time information in the bio-signal as amplitude information of the characteristic points. When the characteristic points such as the time information, the amplitude information, and the like are acquired, a feature used for detecting bio-information may be extracted based on the acquired characteristic points or a combination thereof.

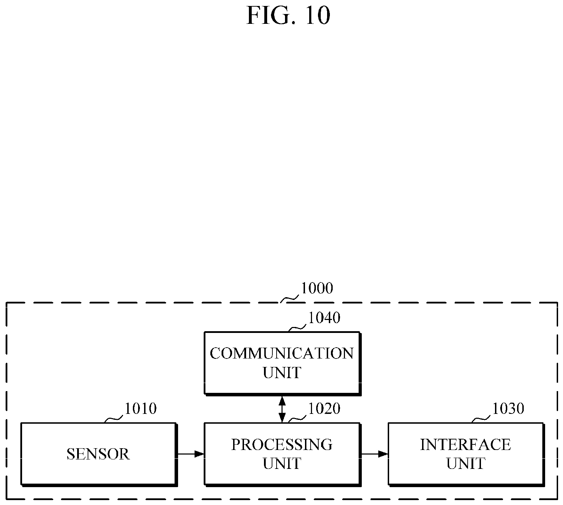

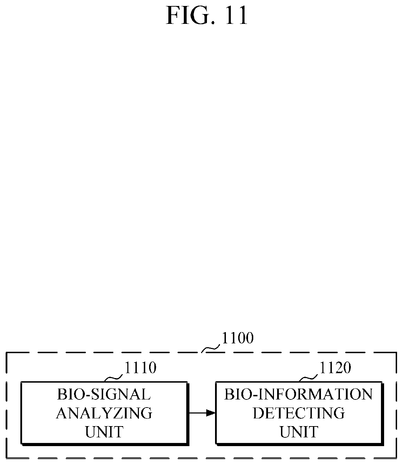

FIG. 10 is a block diagram illustrating an apparatus for detecting bio-information according to an exemplary embodiment, and FIG. 11 is a detailed block diagram illustrating a processing unit of an apparatus for detecting bio-information according to an exemplary embodiment.

An apparatus 1000 for detecting bio-information may be an apparatus for measuring bio-information of a subject, such as blood pressure, in which various exemplary embodiments of the above-described apparatus 100 for extracting a feature of a bio-signal are mounted. For example, the apparatus 1000 for detecting bio-information may be a cuffless type-indirect blood pressure measuring apparatus. The apparatus 1000 for detecting bio-information may be implemented in the form of a wearable device which can be worn by the subject. For example, the apparatus 1000 for detecting bio-information may be implemented in the form of a wristwatch, a bracelet, and a wrist band. However, the apparatus 1000 for detecting bio-information is not limited thereto and can be implemented in the form of a ring, glasses, a hair band, or the like.

Hereinafter, for convenience of description, bio-information to be detected and a bio-signal to be measured will be described as being blood pressure and a pulse wave signal, respectively, although exemplary embodiments are not limited thereto.

Referring to FIG. 10, the apparatus 1000 for detecting bio-information may include a sensor 1010, a processing unit 1020 (e.g., processor), a display unit 1030 (e.g., display), and a communication unit 1040 (e.g., communicator).

When a predetermined control signal is received, the sensor 1010 may irradiate a subject with light by driving a light source and measure a pulse signal by detecting light reflected or scattered from the subject. According to an exemplary embodiment, the subject may be a living body area that can be in contact with or adjacent to the sensor 1010, as a bio-information detecting target and may be a portion of the human body in which a pulse wave can be easily measured through PPG (photoplethysmography). For example, the subject may be a surface area of the wrist adjacent to the radial artery. When a pulse wave is measured on the skin surface of the wrist through which the radial artery passes, this technique may be affected relatively less by external factors that cause error in a measurement such as a thickness of the skin tissue inside the wrist, or the like. The radial artery has been known to correspond to a blood vessel with which accurate blood pressure can be measured compared to other blood vessels inside the wrist. However, the subject is not limited to being a surface area of the wrist adjacent to the radial artery and may be a distal end portion of the human body such as a finger, toe, or the like which has high vascular density.

The processing unit 1020 may receive a control command received from a user through an interface module mounted in the apparatus 1000 for detecting bio-information and process an operation corresponding to the control command. At this point, the interface module may be a display, a microphone, a speaker, a haptic device, or the like which is mounted in the apparatus 1000 for detecting bio-information, but is not limited thereto. The processing unit 1020 may perform various functions for performing interaction with the user through the interface module. As an example, a graphical user interface may be displayed on the display so that the user may input the control command by touching the display. As another example, a conversation agent function and a voice recognition function may be mounted so that the user may perform interaction such as an input of the control command using voice through the microphone, the speaker, or the like.

When the user inputs a control command for requesting detection of bio-information, the processing unit 1020 may generate a signal for controlling the sensor 1010 and transmit the generated signal to the sensor 1010.

When a pulse wave signal is measured by the sensor 1010, the processing unit 1020 may analyze the pulse wave signal in every constant-interval unit and extract a feature used for detecting the blood pressure. For example, the processing unit 1020 may analyze concavity and convexity of the pulse wave signal in every constant-interval unit to extract characteristic points such as time information or amplitude information from the pulse wave signal and extract a feature using the extracted characteristic points.

Referring to FIG. 11, a processing unit 1100 may include a bio-signal analyzing unit 1110 (e.g., bio-signal analyzer) and bio-information detecting unit 1120 (e.g., bio-information detector).

The bio-signal analyzing unit 1110 may acquire a secondary differential signal by secondarily differentiating the pulse wave signal measured by the sensor 1010. In addition, when the secondary differential signal is acquired, the bio-signal analyzing unit 1110 may segment the secondary differential signal in every constant-interval unit, and analyze concavity and convexity of the waveform in every segmented-interval unit. At this point, the number of intervals to be segmented may be set in advance to be larger than or equal to the number of characteristic points desired to be extracted in order to detect blood pressure. In addition, the bio-signal analyzing unit 1110 may adaptively adjust the number of intervals to be segmented according to the type of the waveform.

For example, the bio-signal analyzing unit 1110 may segment the secondary differential signal into constant intervals based on a local maximum point having a locally convex form (upward convex) and a local minimum point having a locally concave form (downward convex) in the secondary differential signal. The bio-signal analyzing unit 1110 may merge two or more segmented intervals in consideration of a fluctuation of the waveform of the secondary differential signal. The bio-signal analyzing unit 1110 may calculate a difference between a secondary differential value of a local maximum point of a current interval and a secondary differential value of a local minimum point of a previous interval as the fluctuation and merge the current interval into the previous interval when the fluctuation is smaller than a preset reference value.

The bio-signal analyzing unit 1110 may calculate a CCD value corresponding to a difference between a secondary differential value of a local maximum point of each interval and a secondary differential value of a local minimum point thereof and select intervals from which characteristic points are to be extracted in descending order of the CCD values. The bio-signal analyzing unit 1110 may obtain time information of the interval corresponding to the local minimum point in the selected intervals and amplitude information of a position corresponding to the time information from a pulse wave signal.

When one or more characteristic points such as the time information or the amplitude information is acquired, the bio-information detecting unit 1120 may extract a feature used for detecting the blood pressure based on at least one of the acquired characteristic points or a combination thereof and detect the blood pressure using the extracted feature. In addition, the bio-information detecting unit 1120 may monitor a health condition of a user based on the detected blood pressure. When it is determined that there is abnormality in the health condition based on the monitored result, the bio-information detecting unit 1120 may generate additional information such as a degree of risk for warning the user.

Referring again to FIG. 10, the interface unit 1030 may provide the blood pressure detected by the processing unit 1020, the additional information such as the degree of risk, warning information, and the like to the user through an interface module which is mounted in the apparatus 1000 or connected to the apparatus 1000 via wired and wireless communications.

For example, the interface unit 1030 may display blood pressure information or risk information for a health condition on a display and provide the displayed information to the user, and, at this point, may display the corresponding information using colors defined in advance according to a degree of risk as shown in Table 1 below so that the user may easily recognize the health condition. In addition, the interface unit 1030 may display a warning message or the like defined in advance according to a degree of risk on the display.

In addition, the interface unit 1030 may output the blood pressure, the degree of risk, the warning information, and the like in an auditory manner using a speaker. In addition, when a haptic device is mounted in or connected to the apparatus 1000, the interface unit 1030 may provide the degree of risk or the warning information to the user through touch or vibration, or other types of feedback. The blood pressure, the degree of risk, the warning information, and the like may be provided to the user by combining two or more visual and non-visual methods according to the type of the interface module, the performance of the apparatus 1000, the purpose of providing information, and the like.

[Table 1]

| Degree of risk | Colors | Vibration/Pressure strength |
|---|---|---|
| High | Red | Strong |
| Medium | Yellow | Medium |
| Low | Green | Weak |

The communication unit 1040 may transmit and receive a variety of information to and from other external devices by communicating with the other external devices via a wireless communication. According to an exemplary embodiment, the wireless communication may include Bluetooth communication, BLE (Bluetooth low energy) communication, near field communication, WLAN (wireless local area network) communication, Zigbee communication, IrDA (infrared data association) communication, WFD (Wi-Fi Direct) communication, UWB (ultra wideband) communication, Ant+ communication, Wi-Fi communication, and mobile communication, although exemplary embodiments are not limited thereto.

The communication unit 1040 may transmit results processed by the processing unit 1020 to other external devices with higher performance to have the other external devices perform desired operations. For example, the communication unit 1040 may transmit the detected blood pressure or the like to a smart phone, a tablet PC, a desktop PC, a notebook PC, or the like so that a user may determine the processed results through a larger display screen.

In addition, when a user inputs a control command such as detection of blood pressure or the like through an external device such as a smart phone or the like, the communication unit 1040 may receive the control command from the external device, and transmit the received control command to the processing unit 1020.

The processing unit 1020 may store the processed results, for example, the detected blood pressure information, the generated additional information, blood pressure history information, health condition monitoring information, a variety of statistic information, and the like in an external storage device mounted in or connected to the apparatus 1000 for detecting bio-information and manage the stored information. At this point, the storage device may include at least one storage medium of a flash memory type, a hard disk type, a multimedia card micro type, a card type memory (e.g., SD or DX memory), RAM (random access memory), SRAM (static random access memory), ROM (read-only memory), EEPROM (electrically erasable programmable read-only memory), PROM (programmable read-only memory), a magnetic memory, a magnetic disk, and an optical disk, but is not limited thereto.

The apparatus 1000 for detecting bio-information may include a main body, and the above-described components 1010, 1020, 1030, and 1040 may be mounted in the main body.

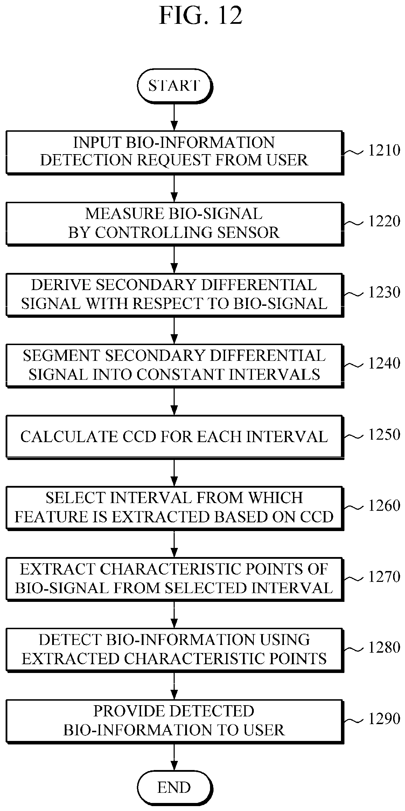

FIG. 12 is a flowchart illustrating a method for detecting bio-information according to an exemplary embodiment.

FIG. 12 shows an exemplary embodiment of a method for detecting bio-information which is performed by the apparatus 1000 for detecting bio-information.

First, in operation 1210, the apparatus 1000 for detecting bio-information may receive a request for detecting bio-information from a user. For example, the user may input a control command for requesting detection of bio-information by operating an operation unit mounted in the apparatus 1000 for detecting bio-information. Alternatively, the apparatus 1000 for detecting bio-information may receive the control command for requesting detection of bio-information from other external devices.

Next, in operation 1220, the apparatus 1000 for detecting bio-information may measure a bio-signal by controlling a sensor according to the control command for requesting detection of bio-information. At this point, the sensor may irradiate a subject with light by driving a light source according to the control signal and detect light reflected or scattered from the subject to measure a bio-signal, for example, a pulse wave.

Next, the apparatus 1000 for detecting bio-information may derive a secondary differential signal by secondarily differentiating the acquired bio-signal in operation 1230, and segment the derived secondary differential signal into constant intervals in consideration of a waveform of the derived secondary differential signal in operation 1240. At this point, the apparatus 1000 for detecting bio-information may adaptively adjust the number of intervals to be segmented in consideration of the waveform of the secondary differential signal while segmenting corresponding intervals based on a local maximum point and a local minimum point of the secondary differential signal. In addition, the apparatus 1000 for detecting bio-information may merge the segmented intervals in consideration of a fluctuation of the waveform as described above.

Next, in operation 1250, the apparatus 1000 for detecting bio-information may calculate a CCD value indicating concavity and convexity of the segmented interval. At this point, the CCD value may be obtained by calculating a difference between secondary differential values of the local maximum point and the local minimum point of each interval.

Next, the apparatus 1000 for detecting bio-information may select intervals corresponding to the number of characteristic points based on the CCD value in operation 1260 and extract a feature of the bio-signal from the selected intervals in operation 1270. According to an exemplary embodiment, the apparatus 1000 for detecting bio-information may extract time information corresponding to a local minimum point of the selected interval and amplitude information of the bio-signal corresponding to the time information as characteristic points and extract a feature used for detecting bio-information through the extracted characteristic points or a combination thereof.

Next, in operation 1280, the apparatus 1000 for detecting bio-information may detect bio-information by utilizing the extracted feature. According to an exemplary embodiment, the apparatus 1000 for detecting bio-information may generate a degree of risk of a user's health condition by analyzing the detected bio-information, for example, blood pressure information.

Next, in operation 1290, the apparatus 1000 for detecting bio-information may provide additional information related to the detected bio-information, the degree of risk to the user, and the like. According to an exemplary embodiment, as described above, the information may be provided to the user by a variety of visual or non-visual methods.

The exemplary embodiments can be implemented as computer readable codes in a computer readable record medium. The computer readable record medium includes all types of recording media in which computer readable data are stored.

Examples of the computer readable recording medium include a ROM, a RAM, a CD-ROM, a magnetic tape, a floppy disk, and an optical data storage. Further, the recording medium may be implemented in the form of a carrier wave (for example, transmission through the Internet). In addition, the computer readable recording medium may be distributed to computer systems over a network, in which computer readable codes may be stored and executed in a distributed manner. Further, a functional program for implementing the disclosures and an associated code and code segment may be easily inferred by programmers in the art to which the disclosures pertains.

A number of examples have been described above. Nevertheless, it will be understood that various modifications may be made. For example, suitable results may be achieved if the described techniques are performed in a different order and/or if components in a described system, architecture, device, or circuit are combined in a different manner and/or replaced or supplemented by other components or their equivalents. Accordingly, other implementations are within the scope of the following claims.

**Claims**

**1.**    A computer-implemented method for extracting a feature from a bio-signal, the method comprising:

acquiring (810) the bio-signal;
deriving a secondary differential signal of the bio-signal;
analyzing (850) concavity and convexity of a waveform of the secondary differential signal; and

extracting (860) the feature from the bio-signal based on the analyzed concavity and the analyzed convexity, **characterized in that**
the analyzing comprises calculating a difference between a local maximum value and a local minimum value in an interval of the secondary differential signal as a convex-to-concave difference, CCD, value, and
the extracting comprises extracting a characteristic point (T1, T2, T3, P1, P2, P3) from the bio-signal based on the calculated CCD value.

2. The method according to claim 1, wherein the method further comprises segmenting the derived secondary differential signal into a preset number of intervals.

3. The method according to claim 2, wherein the extracting of the feature includes selecting the preset number of intervals in descending order of calculated CCD values, and extracting the feature based on a local minimum point of each of the selected intervals.

4. The method according to claim 2, wherein the segmenting comprises segmenting an interval from a local maximum point of the secondary differential signal to a next local minimum point as a single one of the intervals.

5. The method according to claim 2, wherein the segmenting comprises merging two or more segmented intervals based on a difference between a local minimum value and a next local maximum value in the secondary differential signal.

6. The method according to claim 2, wherein the segmenting comprises determining the preset number of the intervals to be segmented in consideration of at least one of complexity of an apparatus, performance of the apparatus, and accuracy of bio-information included in the bio-signal.

7. The method according to claim 2, wherein the segmenting comprises adaptively adjusting the number of the intervals to be segmented according to a type of the waveform of the bio-signal.

8. The method according to one of claims 1 to 7, wherein the bio-signal includes at least one of an electrocardiography, ECG, signal, photoplethysmography, PPG, signal, and an electromyography, EMG, signal.

**Patentansprüche**

1. Computerimplementiertes Verfahren zum Extrahieren einer Eigenschaft aus einem Biosignal, wobei das Verfahren umfasst:

Erfassen (810) des Biosignals;
Ableiten eines sekundären differentiellen Signals des Biosignals;
Auswerten (850) von Konkavität und Konvexität einer Signalform des sekundären differentiellen Signals; und
Extrahieren (860) der Eigenschaft aus dem Biosignal auf der Grundlage der ausgewerteten Konkavität und der ausgewerteten Konvexität,
**dadurch gekennzeichnet, dass**
das Auswerten umfasst: Berechnen einer Differenz zwischen einem lokalen Maximalwert und einem lokalen Minimalwert in einem Intervall des sekundären differentiellen Signals als ein Konvex-zu-Konkav-Differenz-, CCD-, Wert, und
das Extrahieren umfasst: Extrahieren eines charakteristischen Punkts (T1, T2, T3, P1, P2, P3) aus dem Biosignal auf der Grundlage des berechneten CCD-Werts.

2. Verfahren nach Anspruch 1, wobei das Verfahren ferner Segmentieren des abgeleiteten sekundären differentiellen Signals in eine vorgegebene Anzahl an Intervallen umfasst.

3. Verfahren nach Anspruch 2, wobei das Extrahieren der Eigenschaft beinhaltet: Auswählen der vorgegebenen Anzahl an Intervallen in absteigender Reihenfolge berechneter CCD-Werte, und Extrahieren der Eigenschaft auf der Grundlage eines lokalen Minimums jedes der ausgewählten Intervalle.

4. Verfahren nach Anspruch 2, wobei das Segmentieren umfasst: Segmentieren eines Intervalls von einem lokalen Maximum des sekundären differentiellen Signals zu einem nächsten lokalen Minimum als einziges der Intervalle.

**5.** Verfahren nach Anspruch 2, wobei das Segmentieren umfasst: Zusammenführen zweier oder mehrerer segmentierter Intervalle auf der Grundlage einer Differenz zwischen einem lokalen Minimumswert und einem nächsten lokalen Maximumswert in dem sekundären differentiellen Signal.

**6.** Verfahren nach Anspruch 2, wobei das Segmentieren umfasst: Ermitteln der vorgegebenen Anzahl der Intervalle, die zu segmentieren sind, unter Berücksichtigung der Komplexität einer Vorrichtung und/oder des Leistungsvermögens der Vorrichtung und/oder der Genauigkeit der Bioinformation, die in dem Biosignal enthalten ist.

**7.** Verfahren nach Anspruch 2, wobei das Segmentieren umfasst: adaptives Einstellen der Anzahl der Intervalle, die zu segmentieren sind, gemäß einer Art der Signalform des Bio-signals.

**8.** Verfahren nach einem der Ansprüche 1 bis 7, wobei das Biosignal ein Elektrokardiografie-, ECG-, Signal und/oder ein Fotoplethysmografie-, PPG-Signal und/oder ein Elektromyografie-, EMG-Signal umfasst.

**Revendications**

**1.** Procédé implémenté par ordinateur pour l'extraction d'une caractéristique d'un bio-signal, le procédé comprenant :

l'acquisition (810) du bio-signal;
la dérivation d'un signal différentiel secondaire du bio-signal;
l'analyse (850) de la concavité et de la convexité d'une forme d'onde du signal différentiel secondaire ; et
l'extraction (860) de la caractéristique du bio-signal sur la base de la concavité analysée et de la convexité analysée,
**caractérisé en ce que**
l'analyse comprend le calcul d'une différence entre une valeur maximale locale et une valeur minimale locale dans un intervalle du signal différentiel secondaire comme valeur de différence convexe-à-concave, CCD, et l'extraction comprenant l'extraction d'un point caractéristique (T1, T2, T3, P1, P2, P3) à partir du bio-signal sur la base de la valeur CCD calculée.

**2.** Procédé selon la revendication 1, le procédé comprenant en outre la segmentation du signal différentiel secondaire dérivé en un nombre prédéfini d'intervalles.

**3.** Procédé selon la revendication 2, l'extraction de la caractéristique comprenant la sélection du nombre prédéfini d'intervalles en ordre décroissant de valeurs CCD calculées, et l'extraction de la caractéristique sur la base d'un point minimal local de chacun des intervalles sélectionnés.

**4.** Procédé selon la revendication 2, la segmentation comprenant la segmentation d'un intervalle à partir d'un point maximal local du signal différentiel secondaire jusqu'au prochain point minimal local comme l'un unique des intervalles.

**5.** Procédé selon la revendication 2, la segmentation comprenant la fusion de deux intervalles segmentés ou plus sur la base d'une différence entre une valeur minimale locale et une valeur maximale locale suivante dans le signal différentiel secondaire.

**6.** Procédé selon la revendication 2, la segmentation comprenant la détermination du nombre prédéfini des intervalles à segmenter en prenant en considération au moins l'une de la complexité d'un appareil, de la performance de l'appareil, et de la précision de la bio-information incluse dans le bio-signal.

**7.** Procédé selon la revendication 2, la segmentation comprenant l'ajustement de manière adaptative du nombre des intervalles à segmenter en fonction d'un type de la forme d'onde du bio-signal.

**8.** Procédé selon l'une des revendications 1 à 7, le bio-signal comprenant au moins l'un parmi un signal d'ECG d'électrocardiographie, d'un signal PPG de photopléthysmographie, et d'un signal d'EMG d'électromyographie.

FIG. 1

# FIG. 2A

AMPLITUDE

TIME

# FIG. 2B

BIO-SIGNAL → ACQUIRE SECONDARY DIFFERENTIAL SIGNAL → ACQUIRE LOCAL MINIMUM POINT TIME → ACQUIRE TIME AND AMPLITUDE INFORMATION OF BIO-SIGNAL → FEATURE

EP 3 628 215 B1

# FIG. 2C

AMPLITUDE

PPG SIGNAL

$\times 10^{-3}$

P1
P2
P3
P4
DN

TIME

AMPLITUDE

SECONDARY DIFFERENTIAL SIGNAL

$\times 10^{-5}$

$T_2$
$T_3$
$T_1$

TIME

# FIG. 3

```
                              ⌒300
      ┌ ─ ─ ─ ─ ─ ─ ─ ─ ─ ─ ─ ─ ─ ─ ─ ─ ─ ┐
                         ⌒310
      |         ┌──────────────┐          |
                │   INTERVAL   │
BIO-SIGNAL ──── │  SEGMENTING  │          |
      |         │     UNIT     │
                └──────────────┘          |
      |                │
                       │ ⌒320    ⌒330     |
      |         ┌──────▼──────┐ ┌──────────────┐
                │     CCD     │ │   FEATURE    │ |
      |         │ CALCULATING │→│  EXTRACTING  │
                │    UNIT     │ │     UNIT     │ |
      |         └─────────────┘ └──────────────┘
      └ ─ ─ ─ ─ ─ ─ ─ ─ ─ ─ ─ ─ ─ ─ ─ ─ ─ ┘
```

# FIG. 4

AMPLITUDE

PPG SIGNAL

EP 3 628 215 B1

21

# FIG. 5

500

540
SECONDARY DIFFERENTIAL SIGNAL DERIVING UNIT

510
INTERVAL SEGMENTING UNIT

BIO-SIGNAL

520
CCD CALCULATING UNIT

530
FEATURE EXTRACTING UNIT

FIG. 6A

FIG. 6B

FIG. 6C

# FIG. 6D

SELECTED

NOT
SELECTED

# FIG. 6E

FIG. 7A

FIG. 7B

# FIG. 8

```
                    ┌─────────┐
                    │  START  │
                    └────┬────┘
                         │
                         ▼                    810
          ┌──────────────────────────────┐
          │      ACQUIRE BIO-SIGNAL       │
          └───────────────┬──────────────┘
                          │
                          ▼                   820
          ┌──────────────────────────────┐
          │   SEGMENT ACQUIRED BIO-SIGNAL │
          │     INTO CONSTANT INTERVALS   │
          └───────────────┬──────────────┘
                          │
                          ▼          830
                 ╱─────────────────╲        YES    ┌──────────────────────────┐  840
                ╱    ARE SOME        ╲──────────────│   MERGE SOME INTERVALS    │
                ╲ INTERVALS TO BE    ╱              └────────────┬─────────────┘
                 ╲     MERGED?      ╱                            │
                  ╲───────┬───────╱                             │
                          │ NO ◄──────────────────────────────────
                          ▼                   850
          ┌──────────────────────────────┐
          │    ANALYZE CONCAVITY AND      │
          │   CONVEXITY OF BIO-SIGNAL     │
          │  WAVEFORM OF EACH INTERVAL    │
          └───────────────┬──────────────┘
                          │
                          ▼                   860
          ┌──────────────────────────────┐
          │ EXTRACT FEATURE OF BIO-SIGNAL │
          │  BASED ON ANALYZED RESULT     │
          └───────────────┬──────────────┘
                          │
                          ▼
                    ┌─────────┐
                    │   END   │
                    └─────────┘
```

# FIG. 9

START

ACQUIRE BIO-SIGNAL — 910

DERIVE SECONDARY DIFFERENTIAL SIGNAL WITH RESPECT TO ACQUIRED BIO-SIGNAL — 920

SEGMENT SECONDARY DIFFERENTIAL SIGNAL INTO CONSTANT INTERVALS — 930

ARE SOME INTERVALS TO BE MERGED? — 940

YES → MERGE SOME INTERVALS — 950

NO

CALCULATE CCD FOR EACH INTERVAL — 960

SELECT INTERVAL IN DESCENDING ORDER OF CCD — 970

EXTRACT CHARACTERISTIC POINTS FROM SELECTED INTERVAL — 980

EXTRACT FEATURE USING CHARACTERISTIC POINTS — 990

END

# FIG. 10

FIG. 11

1100

1110

1120

| BIO-SIGNAL ANALYZING UNIT | → | BIO-INFORMATION DETECTING UNIT |

# FIG. 12

START

INPUT BIO-INFORMATION
DETECTION REQUEST FROM USER — 1210

MEASURE BIO-SIGNAL
BY CONTROLLING SENSOR — 1220

DERIVE SECONDARY DIFFERENTIAL
SIGNAL WITH RESPECT TO BIO-SIGNAL — 1230

SEGMENT SECONDARY DIFFERENTIAL
SIGNAL INTO CONSTANT INTERVALS — 1240

CALCULATE CCD FOR EACH INTERVAL — 1250

SELECT INTERVAL FROM WHICH
FEATURE IS EXTRACTED BASED ON CCD — 1260

EXTRACT CHARACTERISTIC POINTS OF
BIO-SIGNAL FROM SELECTED INTERVAL — 1270

DETECT BIO-INFORMATION USING
EXTRACTED CHARACTERISTIC POINTS — 1280

PROVIDE DETECTED
BIO-INFORMATION TO USER — 1290

END

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5365428 A